(19) Europäisches Patentamt
European Patent Office
Office européen des brevets

(11) **EP 1 589 438 B1**

(12) **EUROPÄISCHE PATENTSCHRIFT**

(45) Veröffentlichungstag und Bekanntmachung des Hinweises auf die Patenterteilung:
**04.11.2015 Patentblatt 2015/45**

(51) Int Cl.:
**G06F 17/00** *(2006.01)*    **G06F 19/00** *(2011.01)*
**A61B 5/00** *(2006.01)*

(21) Anmeldenummer: **05007701.5**

(22) Anmeldetag: **08.04.2005**

(54) **Verfahren und Vorrichtung zum Überwachen einer Konzentration eines Analyten im lebenden Körper eines Menschen oder Tieres**

Method and device for monitoring concentration of an analyte in a living human or animal body

Procédé et dispositif pour surveillance de la concentration d'un analyte dans un corps vivant humain or animal

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **24.04.2004 DE 102004020160**

(43) Veröffentlichungstag der Anmeldung:
**26.10.2005 Patentblatt 2005/43**

(73) Patentinhaber:
• **Roche Diagnostics GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AT BE BG CH CY CZ DK EE ES FI FR GB GR HU IE IS IT LI LT LU MC NL PL PT RO SE SI SK TR**

(72) Erfinder:
• **Staib, Arnulf, Dr.**
**68723 Oftersheim (DE)**

• **Hegger, Rainer**
**61440 Oberursel (DE)**

(74) Vertreter: **Mommer, Niels et al**
**Twelmeier Mommer & Partner**
**Westliche 56-58**
**75172 Pforzheim (DE)**

(56) Entgegenhaltungen:
**EP-A- 1 102 194    EP-A- 1 518 495**
**US-B1- 6 272 480    US-B1- 6 546 269**

• **SCHREIBER, THOMAS: "Interdisciplinary application of nonlinear time series methods" PHYSICS REPORTS, Bd. 308, 1999, Seiten 1-64, XP002353180**
• **KENNEL, M B; BROWN, R; ABARBANEL, H D I: "Determining embedding dimension for phase-space reconstruction using a geometrical construction" PHYSICAL REVIEW A, Bd. 45, Nr. 6, 1992, Seiten 3403-3411, XP002353181**

EP 1 589 438 B1

**Beschreibung**

Die Erfindung betrifft ein Verfahren und eine Vorrichtung zum Überwachen einer Konzentration eines Analyten im lebenden Körper eines Menschen oder Tieres, bei dem für aufeinanderfolgende Zeitpunkte $t_n$ mit der gesuchten Konzentration korrelierende Analytwerte $y(t_n)$ ermittelt werden.

Ein solches Verfahren ist aus der US 6,546,269 bekannt, wobei ein künftiger Konzentrationswert aus drei früheren Konzentrationswerten mittels einer Taylorentwicklung berechnet wird.

Ein ähnliches Verfahren und eine dafür geeignete Vorrichtung sind auch in der US 5,507,288 beschrieben. Es geht dabei insbesondere um die laufende Überwachung der Konzentration von Glucose im Körper eines Patienten. Sie ist von größter medizinischer Bedeutung. Studien haben zu dem Ergebnis geführt, daß äußerst schwerwiegende langfristige Folgen des Diabetes mellitus (beispielsweise Erblinden aufgrund einer Retinopathie) vermieden werden können, wenn der zeitliche Verlauf der Konzentration der Glucose in vivo kontinuierlich überwacht wird. Dadurch besteht die Möglichkeit, die erforderliche Medikation (Insulin) zu jedem Zeitpunkt exakt zu dosieren und den Blutzuckerspiegel ähnlich wie bei einer gesunden Person ständig innerhalb enger Grenzen zu halten.

Für eine möglichst effiziente Medikation ist es wünschenswert, nicht nur einen momentanen Wert einer Konzentration eines Analyten zu können, sondern auch zukünftige Konzentrationswerte vorhersagen zu kennen. Eine Vorhersage von zukünftigen Werten der Konzentration eines Analyten ist insbesondere bei Diabetes von Bedeutung, um nicht nur einem Anstieg der Blutglucose über einen kritischen Wert hinaus durch Insulingabe entgegenwirken zu können, sondern auch um einen Patienten vor einem Absinken der Blutglucosekonzentration unter einem kritischen Wert und einem damit verbundenen Schwächeanfall zu warnen, so daß dies durch rechtzeitige Kohlenhydrataufnahme verhindert werden kann.

Die Vorhersage einer zukünftigen Konzentration eines Analytwertes im lebenden Körper eines Menschen oder Tieres wird erheblich dadurch erschwert, daß Konzentrationswerte nicht nur von historischen Werten, sondern auch von anderen Parametern wie beispielsweise Nahrungs- und Medikamentaufnahme oder körperlicher Anstrengung abhängt.

Aus der US 6,272,480 B1 ist ein Verfahren zum Überwachen der Blutglucosekonzentration bekannt, bei dem ein künstliches neuronales Netzwerk eingesetzt wird, um aus einer Zeitreihe von gemessenen Glucosekonzentrationen eine Vorhersage für künftige Blutglucosewerte abzuleiten. Ein gravierender Nachteil künstlicher neuronaler Netzwerke liegt jedoch darin, daß diese vor ihrem Einsatz trainiert werden müssen und unzuverlässige Ergebnisse liefern, sobald sie mit Daten einer Zeitreihe konfrontiert werden, die außerhalb des Trainingsbereichs liegen.

Aus der EP 1102194 A2 ist ein Verfahren bekannt, bei dem zu aufeinanderfolgenden Zeitpunkten gemessene Meßwerte der Blutglucosekonzentration unter Einbezug patientenspezifischer Daten, wie insbesondere der Kohlenhydrataufnahme und Insulinabgabe, extrapoliert werden. Für praktische Anwendungen stellt die Notwendigkeit des Erfassens solcher patientenspezifischer Daten einen schweren Nachteil dar, da das Erfassen solcher Daten Mühe bereitet und häufig mit Fehlern behaftet ist.

Aufgabe der Erfindung, welche in Anspruch 1 definiert ist, ist es, einen Weg aufzuzeigen, wie laufend eine Konzentration eines Analyten im lebenden Körper eines Menschen oder Tieres überwacht werden und künftige Konzentrationswerte vorhergesagt werden können. Insbesondere soll dieses Verfahren neben historischen Konzentrationswerten für eine Vorhersage künftiger Konzentrationswerte keine patientenspezifischen Daten wie Nahrungs- oder Insulinaufnahme benötigen.

Diese Aufgabe wird gelöst durch ein Verfahren der eingangs genannten Art, bei dem erfindungsgemäß vorgesehen ist, daß zum Vorhersagen eines Analytwertes $y(t_{n0}+\Delta t)$ über einen Vorhersagezeitraum $\Delta t$ zunächst eine Funktion $F(t_{k,tk-\Delta n,tk-2\Delta n},\cdots,t_{k-(m-2)\Delta n},tk-(m-1)\Delta n)$, die von Analytwerten $y(t_k), y(t_{k-\Delta n}), y(t_{k-2\Delta n}),\ldots, y(t_{k-(m-2)\Delta n}), y(t_{k-(m-1)\Delta n})$ abhängt, bestimmt wird, mit der in einer Nachbarschaft U eines Analytwertes $y(t_{n0})$ zum Zeitpunkt $t_{n0}$ der zeitliche Verlauf der Analytwerte $y(t_n)$ mit einer vorgegebenen Genauigkeit $\sigma$ approximiert werden kann, so daß

$$\sigma^2 \geq \sum_{t_k \in U} [y(t_k) - F(t_k - \Delta t, t_{k-\Delta n} - \Delta t, t_{k-2\Delta n} - \Delta t, \ldots$$

$$\ldots, t_{(k-(m-2)\Delta n)} - \Delta t, t_{(k-(m-1)\Delta n)} - \Delta t)]^2$$

$$(Gl.1),$$

wobei $\Delta n$ eine natürliche Zahl ist und anschließend mittels der Funktion F ein Vorhersagewert

$$y(t_{n0} + \Delta t) = F(t_{n0}, t_{n0-\Delta n}, \dots$$
$$\dots, t_{(n0-(m-2)\Delta n)}, t_{(n0-(m-1)\Delta n)})$$

$$(Gl.2),$$

berechnet wird.

Die Aufgabe wird ferner gelöst durch eine Vorrichtung zum laufenden Überwachen einer Konzentration eines Analyten durch Bestimmung von deren zeitlichem Verlauf im lebenden Körper eines Menschen oder Tieres, mit einer Meßeinheit, durch die zu aufeinanderfolgenden Zeitpunkten $t_n$ Meßwerte einer mit der gesuchten Konzentration korrelierenden Meßgröße als Meßsignal gemessen werden, und mit einer Auswerteeinheit, durch die aus den Meßwerten mit der Konzentration korrelierende Analytwerte $y(t_n)$ ermittelt werden, dadurch gekennzeichnet, daß zum Vorhersagen eines Analytwertes $y(t_{n0}+\Delta t)$ über einen Vorhersagezeitraum $\Delta t$ zunächst eine Funktion F $(t_{k,tk-2\Delta n},\dots,t_{k-2\Delta n},\dots,t_{k-(m-2)\Delta n}, t_{k-(m-1)\Delta n})$, die von Analytwerten $y(t_k)y(t_{k-\Delta n})$, $y(t_{k-2\Delta n}),\dots, y(t_{k-(m-2)\Delta n})$, $y(t_{k-(m-1)\Delta n})$ abhängt, bestimmt wird, mit der in einer Nachbarschaft U eines Zeitpunktes $t_{n0}$ der zeitliche Verlauf der Analytwerte $y(t_n)$ mit einer vorgegebenen Genauigkeit $\sigma$ approximiert werden kann, so daß

$$\sigma^2 \geq \sum_{t_k \in U} [y(t_k) - F(t_k-\Delta t, t_{k-\Delta n} - \Delta t, t_{k-2\Delta n} - \Delta t, \dots$$
$$\dots, t_{(k-(m-2)\Delta n)} - \Delta t, t_{(k-(m-1)\Delta n)} - \Delta t)]^2$$

wobei $\Delta n$ eine natürliche Zahl ist und anschließend mittels der Funktion F ein Vorhersagewert

$$y(t_{n0} + \Delta t) = F(t_{n0}, t_{n0-\Delta n}, \dots$$
$$\dots, t_{(n0-(m-2)\Delta n)}, t_{(n0-(m-1)\Delta n)})$$

berechnet wird.

Bei dem erfindungsgemäßen Verfahren wird der lebende Körper eines Menschen oder Tieres mit der Konzentration eines darin befindlichen Analyten mathematisch als ein deterministisches dynamisches System, das möglicherweise durch Rauschen gestört ist, betrachtet. Die zeitliche Entwicklung dieses Systems wird dabei durch eine Phasenbahn in einem Phasenraum beschrieben, dessen Dimension von der Anzahl der unabhängigen Variablen abhängt, welche die gesuchte Konzentration des Analyten beeinflussen. Im Fall von Diabetes sind beispielsweise die Anzahl der zugeführten Broteinheiten und die Menge des verabreichten Insulins solche Variablen, von denen künftige Werte der Blutglucosekonzentration abhängen.

Für praktische Zwecke ist die Anzahl der Variablen, von denen die gesuchte Analytkonzentration abhängt, in der Regel unbekannt und folglich ist auch die Dimension des Phasenraums, in dem sich die zeitliche Entwicklung des Systems durch die Phasenbahn beschreiben läßt, unbekannt.

Die Erfindung macht sich zunutze, daß zur Vorhersage eines Analytwerts nicht die gesamte Phasenbahn des Systems in einem möglicherweise hochdimensionalen Phasenraum bekannt sein muß. Für die Extrapolation eines Punktes auf der Phasenbahn ist lediglich die Kenntnis der Phasenbahn in einer Umgebung dieses Punktes erforderlich. Bei der Extrapolation muß insbesondere nicht der gesamte Phasenraum des Systems berücksichtigt werden. Es genügt, die Dynamik in einem künstlichen, rekonstruierten Phasenraum von wesentlich geringerer Dimension zu betrachten.

Die Dimension des rekonstruierten Phasenraums muß lediglich so groß sein, daß alle dominierenden Freiheitsgrade, welche die Dynamik des Systems treiben, erfaßt sind. Freiheitsgrade, welche auf die Dynamik des Systems nur vernachlässigbaren Einfluß haben, können unberücksichtigt bleiben, so daß sich die Dimension des rekonstruierten Phasenraums entsprechend kleiner wählen läßt.

Vernachlässigbare Freiheitsgrade treten insbesondere dann auf, wenn ein dynamisches System vorliegt, in dem ein Subsystem schwach an das restliche System ankoppelt. Bei Diabetis beispielsweise ist ein Subsystem, das von der aufgenommenen Insulinmenge, Broteinheiten und körperlicher Bewegung abhängt, schwach mit hormonellen Regelmechanismen gekoppelt, die ebenfalls den Blutzuckergehalt beeinflussen.

In einer Zeitreihe aus den für aufeinanderfolgende Zeitpunkte tn ermittelten Analytwerten $y(t_n)$ ist im Grunde die gesamte Information über das dynamische Verhalten des Systems enthalten. Aus einer endlichen Anzahl von für aufeinanderfolgende Zeitpunkte tn ermittelten Analytwerten - oft genügen bereits etwa 10 Analytwerte in der Umgebung des zu extrapolilierenden Punktes - läßt sich deshalb eine lokale Approximation der Phasenbahn in der Umgebung eines Analytwertes $y(t_{n0})$ zum Zeitpunkt tno ermitteln und um einen Vorhersagezeitraum $\Delta t$ in die Zukunft extrapolieren, so daß ein Vorhersagewert eines Analytwerts $y(t_{n0}+\Delta t)$ berechnet werden kann.

Wichtig ist, daß die Analytwerte, mit denen die Funktion F und damit eine lokale Approximation der Phasenbahn ermittelt wird, nicht für zu dicht aufeinanderfolgende Zeitpunkte tn ermittelt wurden. Andernfalls besteht die Gefahr, daß eine beobachtete zeitliche Entwicklung vorwiegend auf Meßfehlern und Rauscheffekten beruht und nicht die zeitliche Dynamik einer Konzentrationsänderung des Analyten widerspiegelt.

Um durch statistische Methoden den Einfluß unvermeidlicher Meßfehler weitgehend reduzieren zu können, ist es erstrebenswert, Analytwerte $y(t_n)$ für in möglichst kurzen Zeitabständen aufeinanderfolgende Zeitpunkte $t_n$ zu ermitteln. Deshalb kann es notwendig sein, die zur Bestimmung einer lokalen Approximation der Phasenbahn nicht alle für unmittelbar aufeinanderfolgende Zeitpunkte $t_n$ ermittelten Analytwerten $y(t_n)$, sondern nur jeden zweiten, dritten oder n-ten ermittelten Analytwert zu verwenden.

Aus diesem Grund tritt in Gleichung 1 ein $\Delta n$ auf, das dafür sorgt, daß die zur Bestimmung der Funktion F berücksichtigten Analytwerten $y(t_n)$ jeweils für Zeitpunkte $t_n$ bestimmt wurden, die in hinreichenden Zeitabständen aufeinanderfolgen.

Ob mittels einer ermittelten Funktion F der zeitliche Verlauf der Analytwerte $y(t_n)$ mit einer vorgegebenen Genauigkeit approximiert werden kann, läßt sich anhand der Gleichung 1 überprüfen. Dabei ist grundsätzlich über alle Zeitpunkte $t_k$ aus der Nachbarschaft U des Analytwertes $y(t_{n0})$ zum Zeitpunkt $t_{n0}$ zu summieren. Dies schließt aber nicht aus, daß einzelne, beispielsweise durch Anwendung geeigneter Filter, als fehlerhaft anerkannte Analytwerte $y(t_n)$ dabei unberücksichtigt bleiben. Die Funktion F ist erfindungsgemäß eine lineare Funktion, die mit Koeffizienten $a_0$ bis am in der Form

$$ F \ = \ a_0 + \sum_{j=1}^{m} y\left(t_{(k-(m-j)\,\Delta n)}\right) a_j $$

dargestellt wird.

Praktisch lassen sich die Koeffizienten $a_0$ bis $a_m$ dadurch bestimmen, daß die Summe

$$ \sum_{t_k \in U} \left[ y(t_k) \ - \ a_0 \ - \ \sum_{i=1}^{m} y\left(t_{(k-(m-i)\,\Delta n)} - \Delta t\right) a_i \right]^2 $$

minimiert wird. Die Nachbarschaft U ist dabei so zu wählen, daß in ihr hinreichend viele Zeitpunkte $t_n$ für eine Bestimmung der Koeffizienten $a_0$ bis am enthalten sind.

Das erfindungsgemäße Verfahren kann in mehreren Schritten angewendet werden, um aus einem Vorhersagewert $y(t_{n0}+\Delta t)$ einen noch weiter in der Zukunft liegenden Vorhersagewert $y(t_0+2\Delta t)$ zu ermitteln. Durch eine mehrfache Anwendung des Verfahrens läßt sich auf diese Weise ein Vorhersagewert $y(t_0+n\cdot\Delta t)$ ermitteln. Wichtig ist dabei, daß die Koeffizienten $a_0$ bis am, mit deren Hilfe der zeitliche Verlauf der Analytwerte in der Nachbarschaft U approximiert wird, bei jedem Schritt für eine um den Vorhersagezeitraum $\Delta t$ verschobene Nachbarschaft U neu bestimmt werden.

Anstatt durch eine mehrfache Anwendung des Verfahrens in mehreren Schritten einen Analytwert $y(t_n)$ für einen in der Zukunft liegenden Zeitpunkt $t_n$ vorherzusagen, ist es auch möglich, durch Verwendung eines entsprechend größeren Vorhersagezeitraums $\Delta t$ und entsprechend bestimmte Koeffizienten $a_0$ bis $a_m$ in einem einzigen Schritt einen weit in der Zukunft liegenden Analytwert $y(t_n+\Delta t)$ vorherzusagen. Es hat sich aber gezeigt, daß eine schrittweise Anwendung des Verfahrens über größere Zeiträume eine präzisere Vorhersage erlaubt.

Der Erkenntnis, daß sich das dynamische Verhaltens eines deterministischen Systems durch eine endliche Anzahl von für aufeinanderfolgende Zeitpunkte $t_n$ ermittelten Analytwerten $y(t_n)$ erfassen läßt, liegt die in dem Artikel von T. Schreiber in Phys. Rep. 85, 1-64 (1999) beschriebene "Takens time delay embedding" Methode zugrunde. Der Abstand zwischen aufeinanderfolgenden Zeitpunkten, für welche die Analytwerte $y(t_n)$ ermittelt wurden, wird in der Terminologie des Artikels von T. Schreiber als delay time bezeichnet. Auf den Artikel von T. Schreiber wird ergänzend in dem Sinne Bezug genommen, daß dessen Inhalt durch Bezugnahme zum Inhalt der vorliegenden Anmeldung gemacht wird.

Die Erfindung einschließlich bevorzugter Ausführungsformen wird nachfolgend anhand der Figuren näher erläutert. Die darin dargestellten und nachfolgend beschriebenen Besonderheiten können einzeln oder in Kombination verwendet werden, um bevorzugte Ausgestaltungen der Erfindung zu schaffen. Es zeigen:

Fig. 1    ein Blockdiagramm eines erfindungsgemäßen Geräts;

Fig. 2    eine Prinzipskizze eines für die Erfindung geeigneten Sensors;

Fig. 3    ein Meßsignal eines Sensors gemäß Fig. 2;

Fig. 4    gemessene und über einen Zeitraum von 30 Minuten vorhergesagte Blutglucosekonzentrationswerte und

Fig. 5    ein schematisches Beispiel eines Phasenraumes mit Datenpunkten.

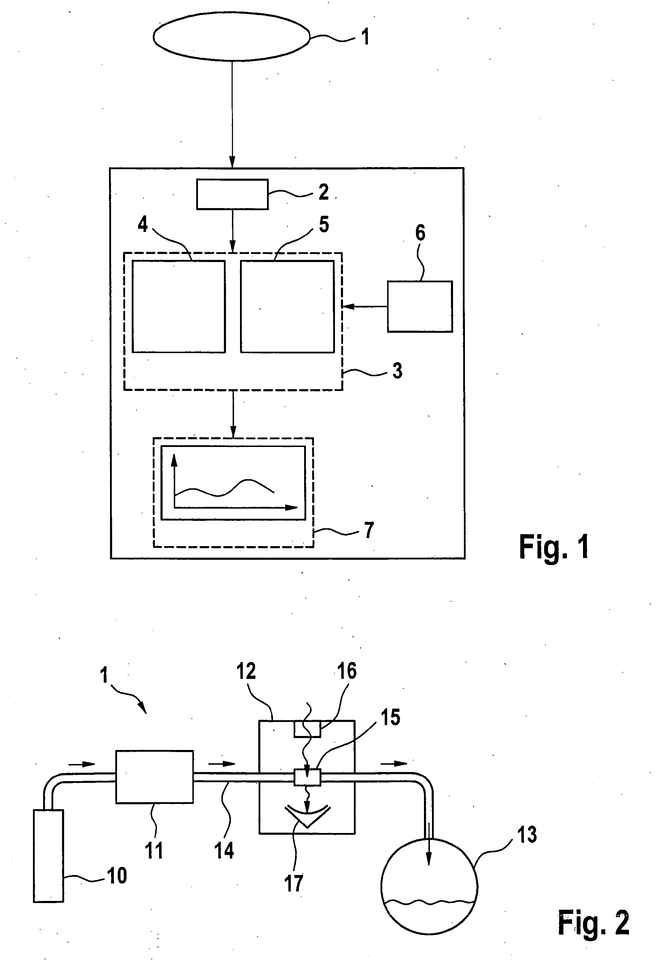

In Fig. 1 sind die wesentlichen Komponenten eines erfindungsgemäßen Geräts dargestellt. Ein Sensor 1 mißt zu aufeinanderfolgenden Zeitpunkten $t_n$ Meßwerte. Dieses Meßsignal wird - im dargestellten Fall drahtlos - an einen Empfänger 2 übertragen, von dem das Meßsignal an eine Auswerteeinheit 3 weitergeleitet wird, die einen Mikroprozessor 4 und einen Datenspeicher 5 enthält. Daten und Befehle können auch über eine Eingabeeinheit 6 an die Auswerteeinheit 3 übermittelt werden. Die Ausgabe von Resultaten erfolgt mittels einer Ausgabeeinheit 7, die ein Display und andere übliche Ausgabemittel einschließen kann. Selbstverständlich erfolgt die Datenverarbeitung in der Auswerteeinheit 3 digital, und es sind entsprechende Wandler zur Umwandlung analoger Signale in digitale Signale vorgesehen. Nähere Erläuterungen hierzu sind nicht erforderlich, weil der grundsätzliche Aufbau derartiger Geräte (beispielsweise aus der US 5,507,288) bekannt ist und die Erfindung zu ganz unterschiedlichen Meßtechniken geeignet ist, bei denen - wie einleitend erläutert - unterschiedliche, mit dem gewünschten Nutzsignal korrelierende Meßsignale gemessen werden.

Fig. 2 zeigt in Form einer Prinzipskizze einen Sensor 1, bei dem ein implantierbarer Katheter 10 verwendet wird, um aus dem Unterhautfettgewebe interstitielle Flüssigkeit mittels einer Pumpe 11 abzusaugen und durch eine photometrische Meßeinheit 12 in einen Abfallbehälter 13 zu saugen. Die Leitung 14, durch die die interstitielle Flüssigkeit transportiert wird, enthält in der photometrischen Meßeinheit 12 eine transparente Meßzelle 15, in die von einem Lichtsensor 16 ausgehendes Primärlicht eingestrahlt wird. Das nach Passieren der Meßzelle 15 resultierende Sekundärlicht wird mittels eines Photodetektors 17 gemessen und mittels einer nicht dargestellten Meßelektronik zu einem Rohsignal verarbeitet, das - wie in Fig. 1 beispielhaft dargestellt - an eine Auswerteeinheit 3 weitergeleitet wird. Mit der Auswerteeinheit 3 werden aus dem Rohsignal Analytwerte $y(t_n)$ ermittelt, die für aufeinanderfolgende Zeitpunkte $t_n$ mit der gesuchten Konzentration korrelieren. Im Rahmen des Ausführungsbeispiels stellen diese Analytwerte $y(t_n)$ den Blutglucosegehalt eines Probanden zu einem Zeitpunkt $t_n$ dar.

Selbstverständlich können Analytwerte $y(t_n)$ auch mit anderen Methoden bestimmt werden, beispielsweise beruhend auf einer enzymatischen Umsetzung von Glucose mit nachfolgendem elektrochemischen Nachweis von dabei gebildetem Wasserstoffperoxid.

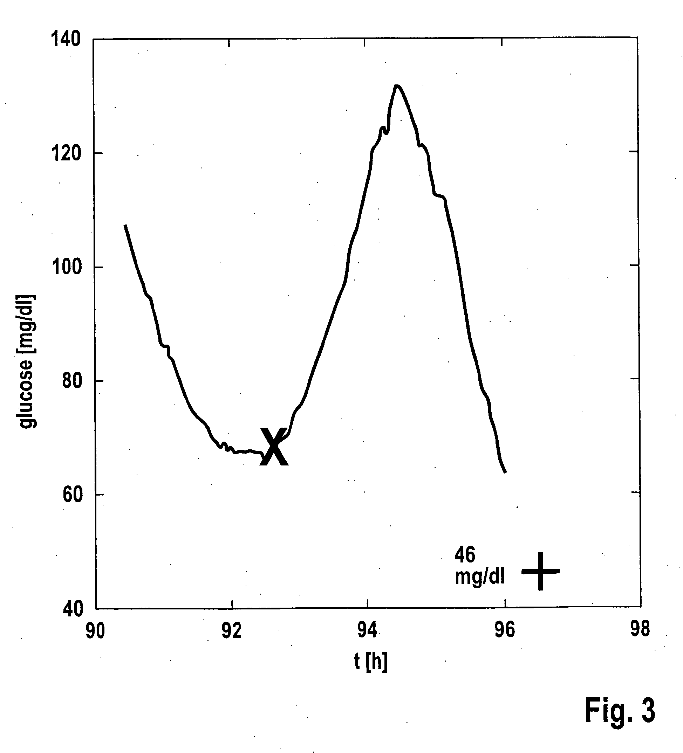

Fig. 3 zeigt als Kurve den zeitlichen Verlauf des Blutglucosegehalts eines Probanden. Die beiden in Fig. 3 eingezeichneten Kreuze stellen exemplarisch jeweils einen Vorhersagewert dar, der mittels des im nachfolgenden beschriebenen Verfahrens über einen Vorhersagezeitraum von 30 Minuten ermittelt wurde. Wie die gute Übereinstimmung des linken Kreuzes mit der durchgezogenen Kurve zeigt, treten die vorhergesagten Werte mit guter Genauigkeit ein. Dies gilt selbstverständlich nur, falls während des Vorhersageintervalls nicht durch eine Insulingabe oder Kohlenhydrataufnahme auf den Blutglucosegehalt eingewirkt wird. Das rechte Kreuz in Fig. 3 zeigt einen Vorhersagewert, der unterhalb eines kritischen Schwellenwerts liegt. Bei einem dermaßen niedrigen Vorhersagewert wird ein Warnsignal erzeugt, um einen Patienten vor einem gefährlichen Absinken des Blutglucosegehalts zu warnen, so daß dem Absinken durch Aufnahme von Kohlenhydraten entgegengewirkt werden kann.

Die in Fig. 3 als Kurve gezeigten Analytwerte $y(t_n)$ wurden aus Meßwerten ermittelt, die im Abstand von jeweils 1 Sekunde aufgenommen wurden. Eine derart hohe Meßrate hat den Vorteil, daß für eine statistische Aufbereitung der Meßwerte genügend Daten zur Verfügung stehen, ist aber nicht unbedingt erforderlich. Für viele Anwendungen ist es ausreichend, wenn im Abstand von wenigen Minuten, beispielsweise 2 bis 5 Minuten, Meßwerte ermittelt werden.

Bei dem gezeigten Ausführungsbeispiel wurden die Meßwerte mittels eines Filteralgorithmus aufbereitet, der eine Operation einschließt, bei der der Einfluß eines aktuellen Meßwerts auf das Nutzsignal mittels eines Gewichtungsfaktors gewichtet wird ("steuerbarer Filteralgorithmus"), wobei auf Basis von während der laufenden Überwachung in engem zeitlichem Zusammenhang mit der Messung des aktuellen Meßwerts detektierten Signalschwankungen ein Signalschwankungsparameter (jeweils bezogen auf den aktuellen Zeitpunkt, d.h. zeitlich veränderlich) ermittelt wird und der Gewichtungsfaktor dynamisch in Abhängigkeit von dem für den Zeitpunkt der aktuellen Meßwert ermittelten Signalschwankungsparameter adaptiert wird. Derartige Filteralgorithmen sind als Kalmann Filteralgorithmen bekannt und beispielsweise in der DE 10343863.7 beschrieben, deren Inhalt durch die Bezugnahme zum Inhalt der vorliegenden Anmeldung gemacht wird.

Für die Zwecke des nachfolgend beschriebenen Verfahrens ist eine aufwendige statistische Aufbereitung der Meßwerte nicht unbedingt erforderlich. Beispielsweise ist es auch möglich, zeitlich über mehrere Meßwerte zur Gewinnung eines Analytwertes $y(t_n)$ zu mitteln oder auch aus jedem Meßwert genau einen Analytwert $y(t_n)$ zu gewinnen.

Mit den ermittelten Analytwerten $y(t_n)$ wird zunächst eine Funktion $F(t_{k,tk-\Delta n}, t_{k-2\Delta n}, ..., t_{k-(m-2)\Delta n}, t_{k-(m-1)\Delta n})$, die von Analytwerten $y(t_k)$, $y(t_{k-\Delta n})$, $y(t_{k-2\Delta n})$, ....., $y(t_{k-(m-2)\Delta n})$, $y(t_{k-(m-1)\Delta n})$ abhängt, bestimmt, die den zeitlichen Verlauf der Analytwerte in einer Nachbarschaft U eines Zeitpunkts $t_{n0}$ mit einer vorgegebenen Genauigkeit $\sigma$ approximiert. Obwohl der zeitliche Verlauf der Analytwerte $y(t_n)$ in der Regel nicht linear ist, hat sich gezeigt, daß eine lineare Funktion

$$F = a_0 + \sum_{j=1}^{m} y\left(t_{(k-(m-j)\Delta n)}\right) a_j$$

mit Koeffizienten $a_0$ bis am ausreicht. Der Wert des Parameters m der Funktion $F(t_{k,tk-\Delta n}, t_{k-2\Delta n}, ..., {}_{k-(m-2)\Delta n}, t_{k-(m-1)\Delta n})$ (z.B. in $a_m$ und $t_{k-(m-1)\Delta n}$) gibt die Dimension des Phasenraumes an, in dem das zeitliche Verhalten des Systems approximiert wird.

Die Koeffizienten $a_0$ bis am lassen sich bestimmen, indem für verschiedene Analytwerte $y(t_k)$ aus der Nachbarschaft U die Gleichung

$$\sigma^2 \geq \sum_{t_k \in U} \left[ y(t_k) - a_0 - \sum_{i=1}^{m} y\left(t_{(k-(m-i)\Delta n)} - \Delta t\right) a_i \right]^2$$

minimiert wird. Werden mit einer hohen Meßrate Meßwerte erfaßt und für entsprechend dicht aufeinanderfolgende Zeitpunkte $t_n$ Analytwerte $y(t_n)$ ermittelt, so ist es sinnvoll, in der obengenannten Gleichung zur Bestimmung der Koeffizienten $a_0$ bis am nicht jeden ermittelten Analytwert, sondern beispielsweise nur jeden zweiten oder dritten Analytwert zu berücksichtigen, so daß $\Delta n$ nicht unbedingt die Zahl 1, sondern auch eine andere natürliche Zahl sein kann. Prinzipiell ist in der obengenannten Gleichung über jeden in der Nachbarschaft U des Zeitpunkts $t_{n0}$ enthaltenen Zeitpunkt $t_k$ zu summieren. Dies schließt aber nicht aus, daß beispielsweise durch geeignete Filter als Ausreißer erkannte Analytwerte $y(t_k)$ unberücksichtigt bleiben.

Idealerweise sollten die Zeitpunkte $t_n$, für welche die Analytwerte $y(t_n)$ bestimmt werden, in konstanten Zeitabständen aufeinanderfolgen. Analytwerte in einem lebenden Körper eines Menschen oder Tieres haben aber über längere Zeit eine so hohe Autokorrelation, daß kleinere Abweichungen von einer konstanten Meßrate die Genauigkeit der Vorhersagewerte nur geringfügig beeinträchtigen. So zeigen beispielsweise Konzentrationen von Lactat - im aeroben Bereich - oder Creatinin über mehrere Stunden eine hohe Autokorrelation. Selbst relativ schnell variierende Anayten, wie beispielsweise Glucose, weisen über einen Zeitraum von 30 Minuten einen Autokorrelationskoeffizienten von über 0,8 auf.

Der Wert des Parameters m der Funktion $F(t_k, t_{k-\Delta n}, t_{k-2\Delta n}, ..., t_{k-(m-2)\Delta n}, t_{k-(m-1)\Delta n})$, also die Dimension des relevanten Phasenraums läßt sich nicht allgemein für alle möglichen Fälle angeben, bei denen die Konzentration eines Analyten im lebenden Körper eines Menschen oder Tieres überwacht werden muß. Dies liegt vor allem daran, daß die Konzentration der verschiedenen medizinisch interessanten Analyten von unterschiedlich vielen unabhängigen Variablen abhängt. Im Fall der Blutglucosekonzentration eines Diabeteskranken sind zwei solche unabhängige Variablen bekannt, nämlich die Menge der aufgenommenen Broteinheiten und die Menge des verabreichten Insulins. Allgemein kann man sagen, daß der Parameter nicht größer sein muß, als die doppelte Anzahl der unabhängigen Variablen plus 1. In dem vorliegenden anhand der Fig. 3 erläuterten Ausführungsbeispiel ist deshalb m = 5.

Der Wert des Parameters m entspricht - wie bereits erwähnt - der Dimensionalität eines nach der "Takens time delay embedding"-Methode rekonstruierten Phasenraums, in dem sich das dynamische Verhalten des Systems beschreiben läßt. Für praktische Zwecke hat m in der Regel einen maximalen Wert zwischen 3 und 10, bevorzugt 5 und 8, und kann empirisch bestimmt werden. Eine Möglichkeit, die Dimensionalität des Phasenraums und damit den Wert dieses Parameters zu bestimmen, liefert auch die Methode der sogenannten "False nearest neighbour"-Analyse, die in einem Artikel von M.B. Kennel et al., Phys. Rev. A 45 3403 (1995) beschrieben ist. Auf diesen Artikel wird ergänzend in dem Sinne Bezug genommen, daß dessen Inhalt durch Bezugnahme zum Inhalt der vorliegenden Anmeldung gemacht wird.

Hat man die Koeffizienten $a_0$ bis am bestimmt, so läßt sich daraus ein Vorhersagewert

$$y\left(t_{n0} + \Delta t\right) = a_0 + \sum_{j=1}^{m} y\left(t_{\left(n0-(m-j)\,\Delta n\right)}\right) a_j$$

berechnen. Ein auf diese Weise ermittelter Vorhersagewert wird dann mit einem vorgegebenen unteren und/oder oberen Schwellenwert vergleichen und bei Überschreiten bzw. Unterschreiten desselben ein Warnsignal erzeugt.

Nimmt ein Patient auf ein solches Warnsignal hin Kohlenhydrate auf oder verabreicht sich eine Insulindosis, so dauert es einige Zeit, bis eine solche Kohlenhydrataufnahme oder Insulinabgabe die Blutglucosekonzentration beeinflußt. Deshalb müssen in der Regel Analytwerte vorhergesagt werden, die 10 bis 90 min., bevorzugt 20 bis 40 min. in der Zukunft liegen, so daß hinreichend Zeit bleibt, einem gefährlichen Ansteigen oder Absinken der Analytkonzentration entgegenwirken zu können. Es hat sich gezeigt, daß sich Analytwerte über größere Zeiträume besser vorhersagen lassen, wenn nicht in einem einzigen Schritt ein derartig großer Vorhersagezeitraum (z.B. $\Delta t = 30$ min) gewählt wird, sondern statt dessen das Verfahren in mehreren kleineren Schritten von beispielsweise 5 Minuten angewandt wird. Dabei werden bei jedem weiteren Schritt für einen bereits berechneten Vorhersagewert $y(t_{n0}+\Delta t)$ Koeffizienten $a_0$ bis am ermittelt, mit denen in einer Nachbarschaft U des Analytwertes zum Zeitpunkt $t_{n0}+\Delta t$ der zeitliche Verlauf der Analytwerte $y(t_n)$ approximiert und daraus ein weiterer Vorhersagewert $y(t_{n0}+2\Delta t)$ berechnet wird. Bei einer solchen schrittweise Vorgehensweise sollte der Vorhersagezeitraum $\Delta t$ der einzelnen Schritte genauso groß wie der Abstand zwischen den Zeitpunkten $t_n$ gewählt werden, für welche die zum Berechnen der Vorhersagewerte verwendeten Analytwerte $y(t_n)$ bestimmt wurden. Bei einem Zeitabstand dt zwischen unmittelbar aufeinanderfolgenden Zeitpunkten $t_n$ bedeutet dies, daß der Vorhersagezeitraum $\Delta t = dt \cdot \Delta n$ ist.

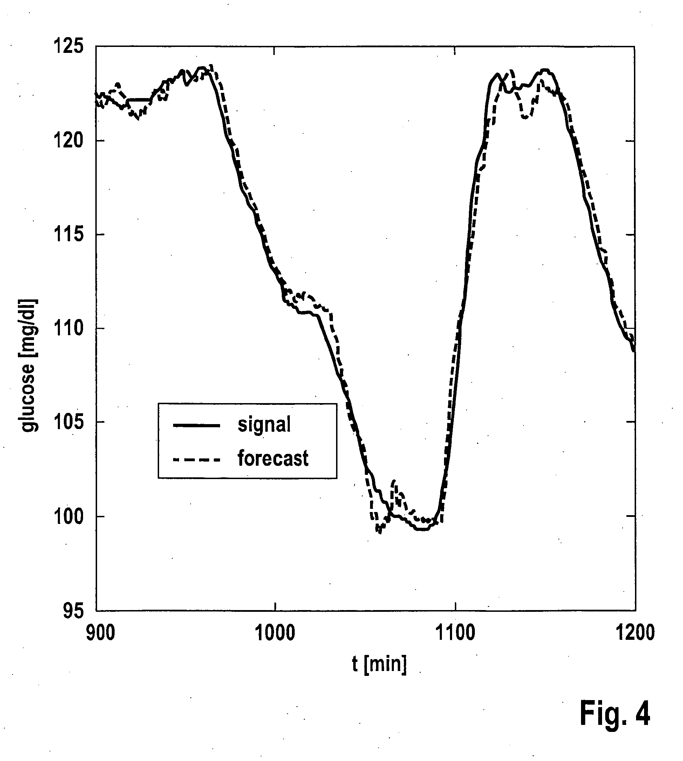

Mit dem beschriebenen Verfahren lassen sich nicht nur zukünftige Analytwerte vorhersagen, sondern es lassen sich auch für die Vergangenheit ermittelte Analytwerte $y(t_n)$ prüfen und auf Ausreißern oder Meßfehlern beruhende falsche Analytwerte $y(t_n)$ identifizieren. Die im allgemeinen sehr gute Übereinstimmung zwischen gemessenen Analytwerten $y(t_n)$ mit Vorhersagewerten zeigt exemplarisch Figur 4. Die glatte, durchgezogene Kurve gibt dabei den zeitlichen Verlauf der Vorhersagewerte, die andere kurzzeitig stark schwankende Kurve den zeitlichen Verlauf der Meßwerte an. Der Vorhersagezeitraum $\Delta t$ betrug dabei 2 min und es wurden aufeinanderfolgend 15 Schritte ausgeführt, so daß Analytwerte über einen Zeitraum von 30 min vorhergesagt wurden. In Figur 4 ist also auf der y-Achse nicht der Blutglucosekonzentration aufgetragen, sondern die Intensität des der Blutglucosebestimmung zugrundeliegenden Meßsignals in Nanoampere bei elektrochemischem Nachweis.

Die Analytwerte $y(t_k),y(t_{k-\Delta n}),y(t_{k-2\Delta n})$, ...., $y(t_{k-(m-2)\Delta n}),y(t_{k-(m-1)\Delta n})$, von denen die Funktion F abhängt, stellen im Sinne der "Takens time delay embedding"-Methode die Koordinaten eines rekonstruierten Phasenraumes dar, in dem das dynamische Verhalten des Systems approximiert und extrapoliert wird. Diese Koordinaten werden auch als Delay-Koordinaten bezeichnet. Rechentechnisch kann es Vorteile haben, wenn die Koordinaten $y(t_k),y(t_{k-\Delta n}),y(t_{k-2\Delta n}),....,y(t_{k-(m-2)\Delta n}),y(t_{k-(m-1)\Delta n})$ transformiert werden und F in Abhängigkeit eines transformierten Koordinatensatzes $Ty(t_k),\ T\,y(t_{k-\Delta n}),...,\ T\,y(t_{k-(m-1)\Delta n})$ bestimmt wird.

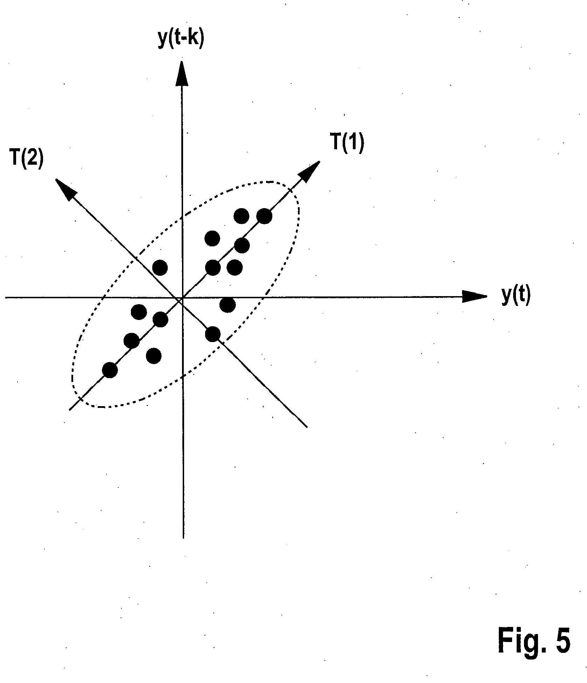

Bevorzugt wird der Koordinatensatz linear transformiert. Dabei ist es günstig, wenn die transformierten Koordinaten sogenannte latente Koordinaten sind, die entlang der Hauptachsen eines sogenannten Varianzellipsoids ausgerichtet sind, wie es in Figur 5 veranschaulicht ist. In Figur 5 ist ein solches Varianzellipsoid in einem zweidimensionalen Phasenraum eingezeichnet, der durch seine Delay-Koordinaten y(t) und y(t-k) dargestellt ist. Das Varianzellipsoid umgibt durch schwarze Kreise dargestellte Datenpunkte, die, wenn sie entsprechend ihrer zeitlichen Abfolge verbunden werden, eine Phasenbahn bilden.

Das Varianzellipsoid läßt sich aus der Streuung der Datenpunkte im Phasenraum berechnen, wobei dessen Hauptachsen die Varianz der Datenpunkte in der jeweiligen Richtung des Phasenraumes angibt. Figur 5 zeigt auch den durch Transformation erhaltenen neuen Phasenraum mit den Koordinaten T(1) und T(2). Die erste latente Koordinate T(1) verläuft entlang der größten Trägheitsachse der Varianzellipsoids, die zweite Koordinate T(2) entlang der nächstgrößeren Trägheitsachse. Numerisch kann eine Transformation in latente Koordinaten, beispielsweise durch sogenannte principal component analysis (M. Otto, Chemometrics, Wiley, Weinheim, 1999, Seite 124 bis 133) oder singular value decomposition (W.H. Press et al., Numerical Recipes, Cambridge University Press, Cambridge, 1989, Seite 52 bis 61) durchgeführt werden.

Durch eine solche Transformation in latente Koordinaten kann erreicht werden, daß eine oder mehrere der transformierten Koordinaten auf die Funktion F innerhalb der vorgegebenen Genauigkeit $\sigma$ vernachlässigbaren Einfluß haben, so daß nur eine entsprechend geringere Anzahl von transformierten Koordinaten bei der Berechnung der Funktion F berücksichtigt werden müssen.

**Patentansprüche**

1. Verfahren zum laufenden Überwachen einer Konzentration eines Analyten durch Bestimmung von deren zeitlichem Verlauf im lebenden Körper eines Menschen oder Tieres, bei dem für aufeinanderfolgende Zeitpunkte $t_n$ mit der gesuchten Konzentration korrelierende Analytwerte $y(t_n)$ ermittelt werden,
wobei zum Vorhersagen eines Analytwertes $y(t_{n0}+\Delta t)$ über einen Vorhersagezeitraum $\Delta t$ zunächst eine lineare Funktion F $(t_k, t_{k-\Delta n}, t_{k-2\Delta n}, \ldots,$
$t_{k-(m-2)\Delta n}, t_{k-(m-1)\Delta n})$, die von Analytwerten $y(t_k), y(t_{k-\Delta n}), y(t_{k-2\Delta n}), \ldots, y(t_{k-(m-2)\Delta n}), y(t_{k-(m-1)\Delta n})$ abhängt, bestimmt wird, mit der in einer Nachbarschaft U eines Analytwertes $y(t_{n0})$ zum Zeitpunkt $t_{n0}$ der zeitliche Verlauf der Analytwerte $y(t_n)$ mit einer vorgegebenen Genauigkeit $\sigma$ approximiert werden kann, so daß

$$\sigma^2 \geq \sum_{t_k \in U} [y(t_k) - F(t_k - \Delta t, t_{k-\Delta n} - \Delta t, t_{k-2\Delta n} - \Delta t, \ldots$$
$$\ldots, t_{(k-(m-2)\Delta n)} - \Delta t, t_{(k-(m-1)\Delta n)} - \Delta t)]^2$$

wobei $\Delta n$ eine natürliche Zahl ist und anschließend mittels der Funktion F ein Vorhersagewert

$$y(t_{n0} + \Delta t) = F(t_{n0}, t_{n0} - \Delta n, \ldots$$
$$\ldots, t_{(n0-(m-2)\Delta n)}, t_{(n0-(m-1)\Delta n)})$$

berechnet wird, **dadurch gekennzeichnet, dass** mit Koeffizienten $a_0$ bis $a_m$ die Funktion F in der Form

$$F = a_0 + \sum_{j=1}^{m} y(t_{(k-(m-j)\Delta n)}) a_j$$

dargestellt wird und die Koeffizienten $a_0$ bis $a_m$ durch Lösen eines linearen Gleichnungssystems bestimmt werden, das für mindestens $m+1$ verschiedene Analytwerte $y(t_k)$ aus der Nachbarschaft U des Zeitpunkts $t_{n0}$ jeweils eine Gleichung

$$y(t_k) = a_0 + \sum_{j=1}^{m} y(t_{(k-(m-j)\Delta n)} - \Delta t) a_j$$

enthält.

2. Verfahren nach Anspruch 1, **dadurch gekennzeichnet, daß** die Funktion F neben den Analytwerten $y(t_k)$, $Y(t_{k-\Delta n}), y(t_{k-2\Delta n}), \ldots, y(t_{k-(m-2)\Delta n}), y(t_{k-(m-1)\Delta n})$ von deren ersten Ableitungen nach der Zeit abhängen.

3. Verfahren nach einem der vorhergehenden Ansprüche, **dadurch gekennzeichnet, daß** die Funktion F neben den Analytwerten $y(t_k), y(t_{(tk-\Delta n)}), y(t_{k-2\Delta n}), \ldots, y(t_{k-(m-2)\Delta n}), y(t_{k-(m-1)\Delta n})$ zusätzlich von deren ersten und zweiten Ableitungen nach der Zeit bestimmt wird.

4. Verfahren nach einem der vorstehenden Ansprüche, **dadurch gekennzeichnet, daß** das Gleichungssystem mehr als m+1 Gleichungen enthält und numerisch nährungsweise zur Bestimmung der Koeffizienten $a_0$ bis am gelöst wird.

**5.** Verfahren nach einem der vorhergehenden Ansprüche, **dadurch gekennzeichnet, daß** die Analytwerte $y(t_n)$ mit denen die Funktion F bestimmt wird, durch numerische Aufbereitung, insbesondere durch Filtern, von Meßwerten gewonnen werden.

**6.** Verfahren nach einem der vorhergehenden Ansprüche, **dadurch gekennzeichnet, daß** in einem weiteren Schritt für einen Vorhersagewert $y(t_{n0}+\Delta t)$ die Funktion F $F(t_k, t_{k-\Delta n}, t_{k-2\Delta n},...,t_{k-(m-2)\Delta n}, t_{k-(m-1)\Delta n})$ an eine Nachbarschaft U eines Analytwertes $y(t_{n0}+\Delta t)$ zu einem Zeitpunkt $t_n = t_{n0}+\Delta t$ angepaßt wird, so daß mit der angepaßten Funktion F der zeitliche Verlauf der Analytwerte $y(t_n)$ in der Nachbarschaft U mit einer vorgegebenen Genauigkeit approximiert und daraus ein weiterer Vorhersagewert $y(t_{n0}+2\Delta t)$ berechnet wird.

**7.** Verfahren nach einem der vorhergehenden Ansprüche, **dadurch gekennzeichnet, daß** die zum Berechnen des Vorhersagewerts verwendeten Analytwerte $y(t_n)$ für in Abständen von 30 Sekunden bis 5 Minuten, vorzugsweise 1 bis 3 Minuten, aufeinanderfolgende Zeitpunkte $t_n$ ermittelt werden.

**8.** Verfahren nach einem der vorhergehenden Ansprüche, **dadurch gekennzeichnet, daß** die zum Berechnen des Vorhersagewerts oder eines der Vorhersagewerte verwendeten Analytwerte $y(t_n)$ für Zeitpunkte $t_n$ ermittelt wurden, die im Abstand des Vorhersagezeitraums $\Delta t$ aufeinanderfolgen.

**9.** Verfahren nach einem der vorhergehenden Ansprüche, **dadurch gekennzeichnet, daß** die Anzahl der Koeffizienten $a_0$ bis am 4 bis 11, bevorzugt 6 bis 9 ist.

**10.** Verfahren nach einem der vorhergehenden Ansprüche, **dadurch gekennzeichnet, daß** F als eine Funktion von Werten $Ty(t_k), Tt(t_{k-\Delta n}), Ty(t_{k-2\Delta n}),...., Ty(t_{k-(m-2)\Delta n}), Ty(t_{k-(m-1)\Delta n})$ bestimmt wird, die durch eine Transformation, vorzugsweise eine lineare Transformation, aus Analytwerten $y(t_k), y(t_{k-\Delta n}), y(t_{k-2\Delta n}),....,y(t_{k-(m-2)\Delta n}), y(t_{k-(m-1)\Delta n})$ bestimmt wurden, wobei die Transformation so gewählt ist, zumindest einer der Werte $Ty(t_k), Ty(t_{k-\Delta n}), Ty(t_{k-2\Delta n}) Ty(t_{k-(m-2)\Delta n}), Ty(t_{k-(m-1)\Delta n})$ in seinem Einfluß auf die Funktion F innerhalb der vorgegebene Genauigkeit $\sigma$ vernachlässigbar ist.

**11.** Vorrichtung zum laufenden Überwachen einer Konzentration eines Analyten durch Bestimmung von deren zeitlichem Verlauf im lebenden Körper eines Menschen oder Tieres,
mit einer Meßeinheit (12), durch die zu aufeinanderfolgenden Zeitpunkten $t_n$ Meßwerte einer mit der gesuchten Konzentration korrelierenden Meßgröße als Meßsignal gemessen werden, und
mit einer Auswerteeinheit (3), durch die aus den Meßwerten mit der Konzentration korrelierende Analytwerte $y(t_n)$ ermittelt werden,
wobei zum Vorhersagen eines Analytwertes $y(t_{n0}+\Delta t)$ über einen Vorhersagezeitraum $\Delta t$ zunächst eine lineare Funktion $F(t_k, t_{k-\Delta n}, t_{k-2\Delta n},..., t_{k-(m-2)\Delta n}, t_{k-(m-1)\Delta n})$, die von Analytwerten $y(t_k)$,
$y(t_{k-\Delta n}), y(t_{k-2\Delta n}),....,y(t_{k-(m-2)\Delta n}), y(t_{k-(m-1)\Delta n})$ abhängt, bestimmt wird, mit der in einer Nachbarschaft U eines Analytwertes $y(t_{n0})$ zum Zeitpunkt $t_{n0}$ der zeitliche Verlauf der Analytwerte $y(t_n)$ mit einer vorgegebenen Genauigkeit $\sigma$ approximiert werden kann, so daß

$$\sigma^2 \geq \sum_{t_k \in U} [y(t_k) - F(t_k - \Delta t, t_{k-\Delta n} - \Delta t, t_{k-2\Delta n} - \Delta t, ... ...,t_{(k-(m-2)\Delta n)} - \Delta t, t_{(k-(m-1)\Delta n)} - \Delta t)]^2 ,$$

wobei $\Delta n$ eine natürliche Zahl ist, und anschließend mittels der Funktion F ein Vorhersagewert

$$y\left(t_{n0} + \Delta t\right) = F\left(t_{n0}, t_{n0 - \Delta n}, \ldots \right.$$
$$\left. \ldots, t_{\left(n0 - (m-2)\Delta n\right)}, t_{\left(n0 - (m-1)\Delta n\right)}\right)$$

berechnet wird, **dadurch gekennzeichnet, daß** mit Koeffizienten $a_0$ bis am die Funktion F in der Form

$$F = a_0 + \sum_{j=1}^{m} y\left(t_{\left(k - (m-j)\Delta n\right)}\right) a_j$$

dargestellt wird und die Koeffizienten $a_0$ bis am durch Lösen eines linearen Gleichnungssystems bestimmt werden, das für mindestens m+1 verschiedene Analytwerte $y(t_k)$ aus der Nachbarschaft U des Zeitpunkts $t_{n0}$ jeweils eine Gleichung

$$y(t_k) = a_0 + \sum_{j=1}^{m} y\left(t_{(k-(m-j)\Delta n)} - \Delta t\right) a_j$$

enthält.

## Claims

1. Method for continuous monitoring of an analyte concentration by determining its progression in the living body of a human or animal, in which analyte values $y(t_n)$ correlating with the concentration to be determined are determined for consecutive points in time $t_n$,
wherein for predicting an analyte value $y(t_{n0}+\Delta t)$ over a prediction period $\Delta t$ a function $F(t_k, t_{k-\Delta n}, t_{k-2\Delta n}, \ldots, t_{k-(m-2)\Delta n}, t_{k-(m-1)\Delta n})$ is determined, which depends on analyte values $y(t_k)$, $y(t_{k-\Delta n})$, $y(t_{k-2\Delta n})$, ..., $y(t_{k-(m-2)\Delta n})$, $y(t_{k-(m-1)\Delta n})$ and which can be used to approximate the progression of the analyte values $y(t_n)$ at time $t_{n0}$ in a vicinity U of an analyte value $y(t_{n0})$ with a predetermined accuracy $\sigma$, such that

$$\sigma^2 \geq \sum_{t_k \in U} \left[ y(t_k) - F\left(t_k - \Delta t, t_{k-\Delta n} - \Delta t, t_{k-2\Delta n} - \Delta t, \ldots \right. \right.$$
$$\left. \left. \ldots, t_{\left(k - (m-2)\Delta n\right)} - \Delta t, t_{\left(k - (m-1)\Delta n\right)} - \Delta t\right)\right]^2$$

whereby $\Delta n$ is an integer, and then by means of a function F a prediction value

$$y\left(t_{n0} + \Delta t\right) = F\left(t_{n0}, t_{n0 - \Delta n}, \ldots \right.$$
$$\left. \ldots, t_{\left(n0 - (m-2)\Delta n\right)}, t_{\left(n0 - (m-1)\Delta n\right)}\right)$$

is calculated, **characterized in that** with coefficients $a_0$ to $a_m$ the function F is represented in the following form

$$F = a_0 + \sum_{j=1}^{m} y\left(t_{\left(k-(m-j)\Delta n\right)}\right) a_j$$

and the coefficients $a_0$ to $a_m$ are determined by solving a system of linear equations which contains one equation each of the type

$$y(t_k) = a_0 + \sum_{j=1}^{m} y\left(t_{(k-(m-j)\Delta n)} - \Delta t\right) a_j$$

for at least m+1 different analyte values $y(t_k)$ from the vicinity U of the point in time $t_{n0}$.

2. Method according to claim 1, **characterized in that** the function F depends on analyte values $y(t_k)$, $y(t_{k-\Delta n})$, $y(t_{k-2\Delta n})$,....,$y(t_{k-(m-2)\Delta n})$, $y(t_{k-(m-1)\Delta n})$ and on their first time derivatives.

3. Method according to any one of the preceding claims, **characterized in that** the function F depends on analyte values $y(t_k)$, $y(t_{k-\Delta n})$,$y(t_{k-2\Delta n})$,....,$y(t_{k-(m-2)\Delta n})$, $y(t_{k-(m-1)\Delta n})$ and on their first and second time derivatives.

4. Method according to any one of the preceding claims, **characterized in that** the system of equations contains more than m+1 equations and is solved numerically by approximation to determine the coefficients $a_0$ to $a_m$.

5. Method according to any one of the preceding claims, **characterized in that** the analyte values $y(t_n)$ used to determine the function F are obtained by numerical processing, in particular by filtering, of measured values.

6. Method according to any one of the preceding claims, **characterized in that** in an additional step the function $F(t_k,t_{k-\Delta n},t_{k-2\Delta n},...,t_{k-(m-2)\Delta n},t_{k-(m-1)\Delta n})$ is adapted in a vicinity U of an analyte value $y(t_{n0}+\Delta t)$ at a point in time $t_n =t_{n0}+\Delta t$ for a calculated prediction value $y(t_{n0}+\Delta t)$, such that the adapted function F approximates the progression of analyte values $y(t_n)$ in the vicinity U with a predetermined accuracy and an additional prediction value $y(t_{n0}+2\Delta t)$ is calculated therefrom.

7. Method according to any one of the preceding claims, **characterized in that** the analyte values $y(t_n)$ used to calculate the prediction value are determined for consecutive points in time $t_n$ which are separated by intervals of 30 seconds to 5 minutes, preferably by 1 to 3 minutes.

8. Method according to any one of the preceding claims claim, **characterized in that** the analyte values $y(t_n)$ used to calculate the prediction value or one of the prediction values are determined for times $t_n$ which are separated by an interval corresponding to the prediction period $\Delta t$.

9. Method according to any one of the preceding claims, **characterized in that** the number of the coefficients, $a_0$ to $a_m$, is 4 to 11, preferably 6 to 9.

10. Method according to any one of the preceding claims, **characterized in that** the function F is determined as a function of values $Ty(t_k)$, $Ty(t_{k-\Delta n})$, $Ty(t_{k-2\Delta n})$,...., $Ty(t_{k-(m-2)\Delta n})$, $Ty(t_{k-(m-1)\Delta n})$, which were determined by means of a transformation, preferably a linear transformation, from analyte values $y(t_k)$,$y(t_{k-\Delta n})$, $y(t_{k-2\Delta n})$,....,$y(t_{k-(m-2)\Delta n})$,$y(t_{k-(m-1)\Delta n})$, whereby the transformation is selected such that at least one of the values $Ty(t_{tk})$, $Ty(t_{k-\Delta n})$, $Ty(t_{k-2\Delta n})$,....,$Ty(t_{k-(m-2)\Delta n})$, $Ty(t_{k-(m-1)\Delta n})$ has a negligible influence on the function F at the predetermined accuracy $\sigma$.

11. Device for continuous monitoring of an analyte concentration by determining its progression in the living body of a human or animal,
comprising a measuring unit (12), by means of which values of a measuring parameter that is correlated with the concentration to be determined are measured as measuring signals at consecutive points in time $t_n$, and
an analytical unit (3), by means of which analyte values $y(t_n)$ that are correlated with the concentration are determined from the measured values, wherein

for predicting an analyte value $y(t_{n0}+\Delta t)$ over a prediction period $\Delta t$ at first a function $F(t_k, t_{k-\Delta n}, t_{k-2\Delta n}, ..., t_{k-(m-2)\Delta n}, t_{k-(m-1)\Delta n})$ is determined, which depends on analyte values $y(t_k)$, $y(t_{k-\Delta n})$, $y(t_{k-2\Delta n})$, ..., $y(t_{k-(m-2)\Delta n})$, $y(t_{k-(m-1)\Delta n})$ and can be used to approximate the progression of the analyte values $y(t_n)$ at time $t_{n0}$ in a vicinity U of an analyte value $y(t_{n0})$ with a pre-determined accuracy $\sigma$, such that

$$\sigma^2 \geq \sum_{t_k \in U} [y(t_k) - F(t_k - \Delta t, t_{k-\Delta n} - \Delta t, t_{k-2\Delta n} - \Delta t, \ldots$$

$$\ldots, t_{(k-(m-2)\Delta n)} - \Delta t, t_{(k-(m-1)\Delta n)} - \Delta t)]^2$$

whereby $\Delta n$ is an integer, and subsequently the function F is used to calculate a prediction value

$$y(t_{n0} + \Delta t) = F(t_{n0}, t_{n0 - \Delta n}, \ldots$$

$$\ldots, t_{(n0 - (m-2)\Delta n)}, t_{(n0 - (m-1)\Delta n)})$$

**characterized in that**
with coefficients $a_0$ to $a_m$ the function F is represented in the following form

$$F = a_0 + \sum_{j=1}^{m} y\left(t_{(k-(m-j)\Delta n)}\right) a_j$$

and the coefficients $a_0$ to $a_m$ are determined by solving a system of linear equations which contains one equation each of the type

$$y(t_k) = a_0 + \sum_{j=1}^{m} y\left(t_{(k-(m-j)\Delta n)} - \Delta t\right) a_j$$

**Revendications**

1. Procédé de surveillance continue d'une concentration d'un analyte par détermination de son parcours temporel dans le corps vivant d'un être humain ou d'un animal, dans lequel des valeurs d'analyte $y(t_n)$ en corrélation avec la concentration recherchée sont déterminées pour des instants $t_n$ successifs,
dans lequel, en vue d'une prédiction d'une valeur d'analyte $y(t_{n0}+\Delta t)$ sur une période de prédiction $\Delta t$, une fonction linéaire $F(t_k, t_{k-\Delta n}, t_{k-2\Delta n}, ..., t_{k-(m-2)\Delta n}, t_{k-(m-1)\Delta n})$, qui dépend de valeurs d'analyte $y(t_k)$, $y(t_{k-\Delta n})$, $y(t_{k-2\Delta n})$, ..., $y(t_{k-(m-2)\Delta n})$, $y(t_{k-(m-1)\Delta n})$, est d'abord déterminée fonction avec laquelle le parcours temporel des valeurs d'analyte $y(t_n)$ peut être approximé avec une précision $\sigma$ prédéfinie à un voisinage U d'une valeur d'analyte $y(t_{n0})$ à un instant $t_{n0}$, de sorte que

$$\sigma^2 \geq \sum_{t_k \in U} [y(t_k) - F(t_k - \Delta t, \quad t_{k-\Delta n} - \Delta t, \quad t_{k-2\Delta n} - \Delta t, \ldots$$

$$\ldots, t_{(k-(m-2)\Delta n)} - \Delta t, t_{(k-(m-1)\Delta n)} - \Delta t)]^2$$

dans lequel $\Delta n$ est un nombre naturel et une valeur de prédiction

$$\mathbf{y}(\mathbf{t}_{n0} + \Delta \mathbf{t}) = \mathbf{F}(\mathbf{t}_{n0}, \mathbf{t}_{n0\text{-}\Delta n}, \ldots$$
$$\ldots, \mathbf{t}_{(n0\text{-}(m\text{-}2)\Delta n)}, \mathbf{t}_{(n0\text{-}(m\text{-}1)\Delta n)})$$

est ensuite calculée au moyen de la fonction F, **caractérisé en ce que** la fonction F est représentée avec des coefficients $a_0$ à $a_m$ sous la forme

$$\mathbf{F} = \mathbf{a}_0 + \sum_{\mathbf{j}=\mathbf{1}}^{\mathbf{m}} \mathbf{y}\big(\mathbf{t}_{(k\text{-}(m\text{-}j)\Delta n)}\big)\mathbf{a}_{\mathbf{j}}$$

et les coefficients $a_0$ à am sont déterminés par résolution d'un système d'équations linéaires qui contient respectivement une équation

$$\mathbf{y}(\mathbf{t}_k) = \mathbf{a}_0 + \sum_{\mathbf{j}=\mathbf{1}}^{\mathbf{m}} \mathbf{y}\big(\mathbf{t}_{(k\text{-}(m\text{-}j)\Delta n)} - \Delta \mathbf{t}\big)\mathbf{a}_{\mathbf{j}}$$

pour au moins m+1 valeurs d'analyte $y(t_k)$ différentes issues de voisinage U de l'instant $t_{n0}$.

2. Procédé selon la revendication 1, **caractérisé en ce que** la fonction F dépend outre des valeurs d'analyte $y(t_k)$, $y(t_{k\text{-}\Delta n})$, $y(t_{k\text{-}2\Delta n})$,..., $y(t_{k\text{-}(m\text{-}2)\Delta n})$, $y(t_{k\text{-}(m\text{-}1)\Delta n})$ de leurs premières dérivées par rapport au temps.

3. Procédé selon l'une quelconque des revendications précédentes, **caractérisé en ce que** la fonction F est déterminée outre pour les valeurs d'analyte $y(t_k)$, $y(t_{k\text{-}\Delta n})$, $y(t_{k\text{-}2\Delta n})$ $y(t_{k\text{-}(m\text{-}2)\Delta n})$, $y(t_{k\text{-}(m\text{-}1)\Delta n})$ de manière supplémentaire à partir de leurs première et seconde dérivées par rapport au temps.

4. Procédé selon l'une quelconque des revendications précédentes, **caractérisé en ce que** le système d'équations contient plus de m+1 équations et est résolu de manière numérique par approximation en vue d'une détermination des coefficients $a_0$ à am.

5. Procédé selon l'une quelconque des revendications précédentes, **caractérisé en ce que** les valeurs d'analyte $y(t_n)$ avec lesquelles est déterminée la fonction F sont obtenues à partir de valeurs de mesures grâce à un traitement numérique, en particulier grâce à un filtrage.

6. Procédé selon l'une quelconque des revendications précédentes, **caractérisé en ce que**, dans une étape ultérieure, la fonction F $F(t_k, t_{k\text{-}\Delta n}, t_{k\text{-}2\Delta n},..., t_{k\text{-}(m\text{-}2)\Delta n}, t_{k\text{-}(m\text{-}1)\Delta n})$ est ajustée à un voisinage U d'une valeur d'analyte $y(t_{n0}+\Delta t)$ à un instant $t_n = t_{n0}+\Delta t$ pour une valeur de prédiction $y(t_{n0}+\Delta t)$, de telle manière que le parcours temporel des valeurs d'analyte $y(t_n)$ est approximé au voisinage U avec une précision prédéfinie grâce à la fonction F ajustée, et une autre valeur de prédiction $y(t_{n0}+2\Delta t)$ est calculée à partir de cela.

7. Procédé selon l'une quelconque des revendications précédentes, **caractérisé en ce que** les valeurs d'analyte $y(t_n)$ utilisées pour le calcul de la valeur de prédiction sont déterminées pour des instants $t_n$ se succédant à des intervalles de 30 secondes à 5 minutes, de manière préférée de 1 à 3 minutes.

8. Procédé selon l'une quelconque des revendications précédentes, **caractérisé en ce que** les valeurs d'analyte $y(t_n)$ utilisées pour le calcul de la valeur de prédiction ou d'une des valeurs de prédiction sont déterminées pour des instants $t_n$ qui se succèdent à un intervalle correspondant à la période de prédiction $\Delta t$.

9. Procédé selon l'une quelconque des revendications précédentes, **caractérisé en ce que** le nombre des coefficients $a_0$ à $a_m$ est de 4 à 11, de manière préférée de 6 à 9.

10. Procédé selon l'une quelconque des revendications précédentes, **caractérisé en ce que** F est définie en tant que fonction de valeurs $Ty(t_k)$, $Ty(t_{k-\Delta n})$, $Ty(t_{k-2\Delta n})$,..., $Ty(t_{k-(m-2)\Delta n})$, $Ty(t_{k-(m-1)\Delta n})$, qui sont déterminées grâce à une transformation, de manière préférée une transformation linéaire, à partir de valeurs d'analyte $y(t_k)$, $y(t_{k-\Delta n})$, $y(t_{k-2\Delta n})$ $y(t_{k-(m-2)\Delta n})$, $y(t_{k-(m-1)\Delta n})$, dans lequel la transformation est choisie de telle manière que l'influence d'au moins une des valeurs $Ty(t_k)$, $Ty(t_{k-\Delta n})$, $Ty(t_{k-2\Delta n})$,..., $Ty(t_{k-(m-2)\Delta n})$, $Ty(t_{k-(m-1)\Delta n})$ sur la fonction F à l'intérieur de la précision $\sigma$ prédéfinie peut être négligeable.

11. Dispositif de surveillance continue d'une concentration d'un analyte par détermination de son parcours temporel dans le corps vivant d'un être humain ou d'un animal,
avec une unité de mesure (12), grâce à laquelle des valeurs de mesure d'une grandeur de mesure en corrélation avec la concentration recherchée sont mesurées en tant que signal de mesure à des instants $t_n$ successifs, et
avec une unité d'évaluation (3), grâce à laquelle des valeurs d'analyte $y(t_n)$ en corrélation avec la concentration sont déterminées à partir des valeurs de mesure,
dans lequel, en vue d'une prédiction d'une valeur d'analyte $y(t_{n0}+\Delta t)$ sur une période de prédiction $\Delta t$, une fonction linéaire $F(t_k, t_{k-\Delta n}, t_{k-2\Delta n},..., t_{k-(m-2)\Delta n}, t_{k-(m-1)\Delta n})$, qui dépend de valeurs d'analyte $y(t_k)$, $y(t_{k-\Delta n})$, $y(t_{k-2\Delta n})$,..., $y(t_{k-(m-2)\Delta n})$, $y(t_{k-(m-1)\Delta n})$, est d'abord déterminée fonction avec laquelle le parcours temporel des valeurs d'analyte $y(t_n)$ peut être approximé avec une précision $\sigma$ prédéfinie à un voisinage U d'une valeur d'analyte $y(t_{n0})$ à un instant $t_{n0}$, de sorte que

$$\sigma^2 \geq \sum_{t_k \in U} [y(t_k) - F(t_{k\text{-}\Delta t}, \qquad t_{k\text{-}\Delta n} - \Delta t, \qquad t_{k\text{-}2\Delta n} - \Delta t, \ldots$$
$$\ldots, t_{(k\text{-}(m\text{-}2)\Delta n)} - \Delta t, t_{(k\text{-}(m\text{-}1)\Delta n)} - \Delta t)]^2$$

dans lequel $\Delta n$ est un nombre naturel, et une valeur de prédiction

$$y(t_{n0+\Delta t}) = F(t_{n0}, t_{n0\text{-}\Delta n}, \ldots$$
$$\ldots, t_{(n0\text{-}(m\text{-}2)\Delta n)}, t_{(n0\text{-}(m\text{-}1)\Delta n)})$$

est ensuite calculée au moyen de la fonction F, **caractérisé en ce que** la fonction F est représentée avec des coefficients $a_0$ à $a_m$ sous la forme

$$F = a_0 + \sum_{j=1}^{m} y(t_{(k\text{-}(m\text{-}j)\Delta n)}) a_j$$

et les coefficients $a_0$ à $a_m$ sont déterminés par résolution d'un système d'équations linéaires qui contient respectivement une équation

$$y(t_k) = a_0 + \sum_{j=1}^{m} y(t_{(k\text{-}(m\text{-}j)\Delta n)} - \Delta t) a_j$$

pour au moins $m+1$ valeurs d'analyte $y(t_k)$ différentes issues du voisinage U de l'instant $t_{n0}$.

**Fig. 1**

**Fig. 2**

Fig. 3

Fig. 4

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6546269 B
- US 5507288 A
- US 6272480 B1
- EP 1102194 A2
- DE 10343863

**In der Beschreibung aufgeführte Nicht-Patentliteratur**

- **T. SCHREIBER.** *Phys. Rep.,* 1999, vol. 85, 1-64
- **M.B. KENNEL et al.** *Phys. Rev.,* 1995, vol. A 45, 3403
- **M. OTTO.** Chemometrics. Wiley, 1999, 124-133
- **W.H. PRESS et al.** Numerical Recipes. Cambridge University Press, 1989, 52-61